# EUROPEAN PATENT APPLICATION

(11) **EP 0 722 704 A1**
(43) Date of publication of application: **24.07.1996**
(21) Application number: 96100721.8
(22) Date of filing: 18.01.1996
(51) Int. Cl.: A61F 5/01, A61F 5/30

(54) **Ankle bandage**

(30) Priority: 19.01.1995 IT PD950015
(71) Applicant: Martella, Pasquale, 35010 Vigodarzere (Padova) (IT)
(72) Inventor: Martella, Pasquale, 35010 Vigodarzere (Padova) (IT)
(74) Representative: Modiano, Guido, Dr.-Ing.

(57) **Abstract**

The invention relates to a device for therapies for the repression of receptor syndromes caused by traumas or inflammatory processes affecting the foot or the ankle and/or disorders affecting other regions. The device comprises an ankle band made of elastic fabric with reinforcements at the regions related to the lateral ligaments of the ankle and an internal elastic presser that can be arranged so as to be centered on the inlet of the outer opening of the sinus tarsi.

## Description

The present invention relates to a device for therapies for the repression of receptor syndromes caused by traumas or inflammatory processes affecting the foot or the ankle and/or disorders affecting other regions.

It is known that the sinus tarsi is a funnel-shaped tunnel that divides the anterior subastragalar articulation of the foot from the posterior articulation.

The external inlet is located in a subpremalleolar region and is covered by a pad of fatty tissue.

Lateral stability of the astragalus is provided by the anterior astragalar peroneal ligament, by the posterior astragalar peroneal ligament, by the calcaneal peroneal ligament, and by the outer calcaneal astragalar ligament.

It is also known that the sinus tarsi is a large receptor site of foot proprioception; in other words, it is the seat of a whole plurality of receptors, i.e. of structures which convert various forms of ambient energy into action potential of the sensory nerves.

The receptor site of the sinus tarsi is subject to what can be defined as "receptor syndromes", which can be caused by traumas or by inflammatory processes affecting the foot or the ankle.

In normal conditions, once adequate stimulation has been received, the receptors send the received message, through the posterior root, to the spinal cord, which is organized so as to route them towards the upper centers and provide immediate and adequate motor and/or vegetative responses through a reflex arc.

Each receptor has its own specificity, i.e., it is specialized in responding to specific stimuli.

When the stimuli exceed certain threshold values, the receptors lose their specificity and send pain impulses.

Therefore, if there is an irritation focus, constituted by a trauma or by an inflammatory process that alters the reception signal, this produces a stimulus that reaches the spinal cord, producing an altered response of the motor and sympathetic nervous system, with consequent defensive muscle contracture as well as arteriole and venule constriction.

The muscle contracture affects most of all the dorsal extensor muscles of the foot and the peroneal muscles, whereas the alterations affecting the vessels produce capillary stasis, hyperemia, and an increase in vessel permeability with formation of an oedema.

In the worst cases, the blood flow increases also at the bone level, with an increase in bone turnover, leading to demineralization of the bone structures of the foot.

Infiltrations of carbocaine on the sinus tarsi are currently used to cure disorders constituted by receptor syndromes, and in some cases the disorders tend to decrease or disappear, but a cure is not necessarily achieved and the consequent swelling does not necessarily disappear.

A principal aim of the present invention is to provide a device that can be used for therapies for the repression of receptor syndromes instead of conventional carbocaine infiltrations.

Within the scope of the above aim, a consequent primary object is to provide a device the use whereof leads to an improvement of the results obtained so far with conventional therapies.

Another important object is to provide a device which is structurally simple and can be produced without particular difficulties.

Another object is to provide a device that does not have any particular difficulties in placement.

Another object is to provide a device having a low cost.

This aim, these objects, and others which will become apparent hereinafter are achieved by a device for therapies for the repression of receptor syndromes caused by traumas or inflammatory processes affecting the foot or the ankle and/or disorders affecting other regions, characterized in that it comprises an ankle band made of elastic fabric with reinforcements at the regions related to the lateral ligaments of the ankle and an internal elastic presser that can be arranged so as to be centered on the inlet of the outer opening of the sinus tarsi.

Further characteristics and advantages of the invention will become apparent from the detailed description of an embodiment thereof, illustrated only by way of non-limitative example in the accompanying drawings, wherein:

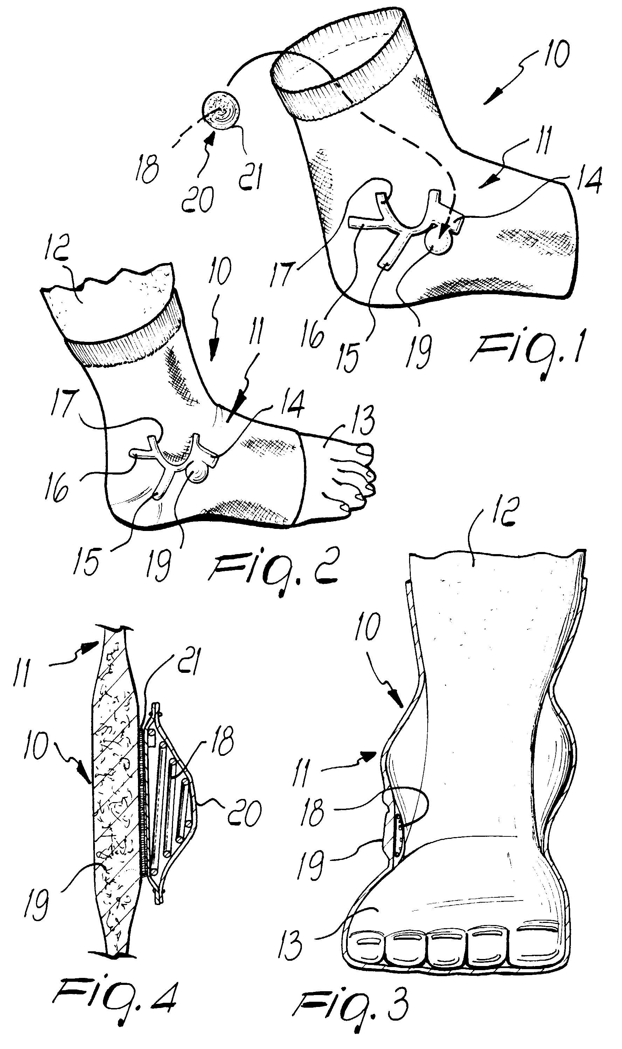

figure 1 is a view of the device, wherefrom an internal elastic presser, included therein, has been extracted;
figure 2 is a view of the device of figure 1 during use;
figure 3 is a sectional view of the device of figure 1 during use;
figure 4 is an enlarged-scale sectional view of a detail of the device.

With reference to the above figures, a device for therapies for the repression of receptor syndromes caused by traumas or inflammatory processes affecting the foot or the ankle and/or disorders affecting other regions, according to the invention, is generally designated by the reference numeral 10 and comprises an ankle band 11, made of elastic fabric, the shape and size whereof are such as to reach from the lower median third of the leg to the metatarsal heads of the foot.

In the figures, the leg is designated by the reference numeral 12 and the foot is designated by the reference numeral 13.

The ankle band 11 is provided with three reinforcements, respectively 14, 15, and 16, also made of elastic fabric, at the regions related to the three lateral ligaments of the ankle.

In particular, a first reinforcement 14, which represents the anterior astragalar peroneal ligament, runs from the lower part of the edge of the peroneal malleolus to the lateral face of the neck of the astragalus, with a slight obliqueness from the top to the bottom and from the rear to the front.

The second reinforcement 15 runs from the tip of the peroneal malleolus to the lateral face of the calcaneum, at the peroneal-calcaneal ligament and at the outer astragalar-calcaneal ligament.

Said second reinforcement runs in an oblique direction from the top to the bottom and from the front to the rear.

The third reinforcement 16 instead follows the direction of the posterior astragalar peroneal ligament and runs from the posterior medial fovea of the peroneal malleolus to the lateral tubercle of the posterior process of the astragalus, with a substantially horizontal orientation.

The first reinforcement 14 and the second reinforcement 15 conveniently form an angle of approximately 95 sexagesimal degrees therebetween.

All the reinforcements 14, 15, and 16 are provided by enlarging the elastic fabric by increasing its threads.

As regards the dimensions of the reinforcements 14, 15, and 16, they can vary according to foot size.

A fourth reinforcement 17 connects the previous ones, wrapping around the malleolus in a downward region.

The-device 10 furthermore comprises an internal elastic presser constituted in practice by a helical spring 18 made of stainless steel with a wire diameter of 1 mm and a frustum-like shape, in which the larger and smaller flat faces have a diameter that is preferably on the order of 20-22 mm and 10 mm respectively; its height is preferably on the order of 12 mm.

The load thus produced is preferably around 1 kg.

Said spring 18 is arranged internally, below the lower part of the first reinforcement 14, and is mounted at a fifth disk-like reinforcement 19 of the same type as the preceding ones, so that the smaller flat face lies toward the skin.

The spring 18 is conveniently combined with a covering 20, preferably made of leather, to avoid direct contact of the metal with the skin, which might cause abrasions and excoriations thereon.

Fixing to the ankle band 11 can be provided by means of a Velcro fixing 21 that allows to adjust the position, since centering of the spring 18 on the inlet of the outer opening of the sinus tarsi is necessary.

The primary feature of the spring is in fact to produce dynamic compression on the skin, on the bouchon that covers the inlet of the sinus tarsi, and on the anterior astragalar-calcaneal ligament.

These structures, which are conventionally rich in receptors, are stimulated by the spring at the free terminations, which are sensitive to pain stimuli, on the touch-sensitive Meissner and Merkel corpuscles present on the epidermis, on the corpuscles of Golgi and on the pacinian corpuscles that are sensitive to light and intense pressures and are present in the hypodermis, on the pacinian corpuscles and the free terminations that are present in the fatty tissue covering the inlet of the sinus tarsi, on the corpuscles of Golgi, and on the free terminations that are present in the anterior astragalar-calcaneal ligament.

The spring 18, by producing a slight painful irritation and a compression that varies according to the dynamics of the step, has the effect of returning to normality the signals that depart from these receptors and of rebalancing the disorder that had occurred.

The presence of the three reinforcements 13, 14, and 15 on the ankle band 11 furthermore allows to stabilize the talocrural and the subastragalar articulation, allowing more correct walking and accordingly allowing again the reception and transmission of adequate stimuli on the part of the receptors.

Practical tests have shown that the use of the device 10 has had beneficial effects in all receptor syndromes caused by traumas or inflammatory processes affecting the foot or the ankle.

In particular in the syndrome affecting the sinus tarsi, complete repression of all symptoms has been observed.

Further testing has furthermore shown the recovery of disorders affecting other regions and linked to alterations of the signals departing from foot receptors.

It has thus been observed that the intended aim and objects of the present invention have been achieved.

The invention thus conceived is susceptible of numerous modifications and variations, all of which are within the scope of the inventive concept.

All the details may furthermore be replaced with other technically equivalent elements.

In practice, the materials employed, so long as they are compatible with the contingent use, as well as the dimensions, may be any according to the requirements.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. Device for therapies for the repression of receptor syndromes caused by traumas or inflammatory processes affecting the foot or the ankle and/or disorders affecting other regions, characterized in that it comprises an ankle band made of elastic fabric with reinforcements at the regions related to the lateral ligaments of the ankle and an internal elastic presser that can be arranged so as to be centered on the inlet of the outer opening of the sinus tarsi.

2. Device according to claim 1, characterized in that said internal elastic presser is constituted by a helical spring made of stainless steel.

3. Device according to claim 2, characterized in that said spring is frustum-shaped.

4. Device according to claims 2 and 3, characterized in that said spring is arranged so that its smaller flat face is directed towards the skin.

5. Device according to claims 2, 3, and 4, characterized in that said spring preferably has dimensions that are on the order of a diameter of 20-22 mm for the larger flat face, a diameter of 10 mm for the smaller flat face, and 12 mm for the height.

6. Device according to claims 2, 3, and 4, characterized in that said spring is sized so as to apply a load of preferably 1 kg.

7. Device according to one or more of the preceding claims, characterized in that said reinforcements are made of elastic fabric.

8. Device according to one or more of the preceding claims, characterized in that a first one of said reinforcements is arranged so as to follow the path of the anterior astragalar peroneal ligament.

9. Device according to one or more of the preceding claims, characterized in that a second one of said reinforcements is arranged so as to follow the path of the peroneal-calcaneal and astragalar-calcaneal ligament.

10. Device according to one or more of the preceding claims, characterized in that a third one of said reinforcements is arranged so as to follow the path of the posterior astragalar peroneal ligament.

11. Device according to one or more of the preceding claims, characterized in that it comprises a disk-like reinforcement at the location of said spring.

12. Device according to one or more of the preceding claims, characterized in that said spring is fixed to said ankle band by means of a Velcro-based fixing.

13. Device according to one or more of the preceding claims, characterized in that said ankle band is configured so as to run from the median lower third of the leg to the metatarsal heads of the foot.
